# NOUVEAU FASCICULE DE BREVET EUROPEEN

(11) **EP 0 688 561 B2**
(45) Date de publication et mention de la décision concernant l'opposition: **26.05.2004**
(45) Mention de la délivrance du brevet: 31.07.1996
(21) Numéro de dépôt: 95401146.6
(22) Date de dépôt: 17.05.1995
(51) Int. Cl.: A61K 7/48

(54) **Gel gommant huileux**
Öliges Peeling Gel
Oily peeling gel

(30) Priorité: 20.06.1994 FR 9407533
(43) Date de publication de la demande: 27.12.1995
(73) Titulaire: L'OREAL, 75008 Paris (FR)
(72) Inventeur: Marion, Catherine, F-92330 Sceaux (FR); Louvet, Nathalie, F-94550 Chevilly Larue (FR)
(74) Mandataire: Dossmann, Gérard

(56) Documents cités:
- CH-A- 678 188
- FR-A- 2 552 099
- US-A- 5 178 881
- PATENT ABSTRACTS OF JAPAN vol. 17, no. 674 (C-1140) 10 Décembre 1993 & JP 05 221 822 A (SHISEIDO CO LTD) 31 Août 1993
- Römpp Chemie Lexikon, 9ième edition, Prof. Dr. Jürgen Falbe, Düsseldorf et Prof. Dr. Manfred Regitz, Kaiserslautern, page 1511
- Le Petit Larousse Illustré, 1993, page 474
- Consumer's Dictionary of Cosmetic Ingredients, Crown Publishers, 1974, p. 106
- "Grundlagen und Rezepturen der Kosmetika", Karlheinz Schrader, 2. Auflage, 1989, Hüthig Buch Verlag GmbH, Heidelberg, pages 939 - 941
- "Formes pharmaceutiques pour application locale", Monique Seiller, Marie-Claude Martini, 1996, Lavoisier TEC DOC, Londres, Paris, New York, pages 25, 26, 82

## Description

L'invention se rapporte à un gel huileux gommant utilisable notamment dans les domaines cosmétique et/ou dermatoiogique pour nettoyer en profondeur la peau du visage, du cou et/ou du corps humain. En particulier, ce gel permet l'élimination des cellules mortes et impuretés de la peau, et le débouchage des pores de la peau tout en douceur.

L'invention a aussi pour objet un procédé pour nettoyer la peau du corps, du cou et/ou du visage.

Les produits gommants ou exfoliants généralement utilisés se présentent sous forme d'émulsions comme dans le document CH-678 488. Ces émulsions contiennent comme particules exfoliantes de la poudre de polyéthylène, de fines particules de quartz ou de coques de noix. Malheureusement, ces produits exfoliants présentent les inconvénients d'être irritants et de nécessiter après leur usage l'application d'une crème nutritive, protectrice et/ou hydratante. Ces produits sont notamment très mal supportés par les peaux sensibles.

L'irritation de ces produits est généralement due à la présence d'agents tensioactifs destinés à stabiliser la dispersion d'une phase dans l'autre (huile-dans-eau ou eau-dans-huile). En outre, ces émulsions provoquent des dessèchements de la peau et sont mal supportées par les peaux très sèches.

Pour remédier à ces inconvénients, le demandeur a envisagé la réalisation d'un gel huileux gommant présentant à la fois des propriétés exfoliantes, et d'hydratation de la peau ainsi qu'une grande douceur et du confort à l'application.

Actuellement, il n'existe pas de produit exfoliant quasi-anhydre, c'est-à-dire contenant moins de 10 % d'eau.

De façon surprenante, le demandeur a trouvé qu'il était possible d'introduire des particules exfoliantes dans un gel huileux, n'irritant pas et ne desséchant pas la peau, tout en assurant un parfait gommage de la peau. En outre, le gel huileux ainsi obtenu ne nécessite pas l'emploi d'une crème nutritive, protectrice et/ou hydratante après usage du gel. Ce dernier est du type produit "2 en 1".

Pour introduire des particules exfoliantes dans un gel huileux, le demandeur a dû déterminer la viscosité du gel pour assurer une dispersion homogène des particules exfoliantes. En effet, lorsque le milieu huileux dans lequel on doit introduire les particules exfoliantes est trop fluide, l'incorporation, de façon homogène, de ces particules est difficile à réaliser. En effet, ces particules ont tendance à flotter au-dessus du milieu huileux.

A l'inverse, lorsque le milieu huileux est trop compact, l'introduction de ces particules exfoliantes ne peut se faire qu'à chaud, ce qui pose un certain nombre de problèmes.

Ainsi, l'invention a pour objet un gel huileux gommant, se caractérisant en ce qu'il contient une dispersion homogène de particules exfoliantes et en ce qu'il présente une viscosité allant de 100 mPa.s à 15 000 mPa.s.

Ce gel correspond à un nouveau concept, associant le gommage et le soin en un seul traitement, contrairement à ce qui se faisait jusqu'à ce jour.

Selon l'invention, ce gel huileux contient moins de 10 % en poids d'eau par rapport au poids total du gel et notamment de 0 % à 5 % d'eau. Les particules exfoliantes ou gommantes utilisables dans l'invention peuvent être d'origine minérale, végétale ou organique. Ainsi, on peut utiliser de la poudre de polyéthylène, de la poudre de nylon, de la poudre de polychlorure de vinyle, des broyais de noyaux d'abricots ou de coques de noix, de la sciure de bois, des billes de verre, etc....

De préférence, on utilise des particules en matériau organique polymérique. Ces particules ont l'avantage d'être moins agressives que les particules d'origine minérale. Avantageusement, ces particules sont des particules de polyéthytène.

Pour obtenir un gommage efficace de la peau, on utilise de préférence des particules ayant un diamètre allant de 100 µm à 500 µm. En outre, pour conférer une plus grande douceur à ce gommage, on utilise avantageusement des particules ayant un diamètre allant de 150 µm à 300 µm.

Contrairement à toute attente, le gel huileux de l'invention ne confère pas à la peau de sensation de gras. Ceci est essentiellement dû à la présence des particules.

Selon l'invention, le gel peut contenir de 50 % à 95 % en poids d'huile par rapport au poids total du gel, et mieux, de 70 % à 85 % en poids d'huile.

La quantité de particules peut varier, selon leur nature et leur taille, de 0,5 % à 15 % en poids par rapport au poids total du gel et, de préférence, être choisie dans la gamme allant de 1 % à 12% en poids.

Les huiles utilisables dans l'invention peuvent être d'origine minérale, végétale, synthétique, siliconée ou fluorée. Ces huiles sont celles classiquement utilisées dans le domaine cosmétique et/ou dermatologique.

A titre d'exemples, on peut citer comme huile minérale, l'huile de paraffine ou de vaseline comme huile végétale, l'huile d'amande douce, d'avocat, de ricin, d'olive, de jojoba, de pépin de raisin, de palme, de germe de blé, le beurre de karité; comme huile synthétique, l'octyldodécanol et les esters gras tels que le myristate de butyle ou d'isopropyle ou le palmitate d'isopropyle comme huile siliconée, les cyclométhicones ou les polydiméthylsiloxanes volatils.

Pour réaliser le gel, il est nécessaire d'utiliser au moins un gélifiant d'huile. Ce gélifiant doit être utilisé en quantité suffisante pour conférer à la composition la viscosité mentionnée ci-dessus. Comme agents gélifiants, on peut utiliser tous ceux classiquement utilisés dans les milieux cosmétiques ou dermatologiques lipophiles. A titre d'exemples, on peut citer la vaseline, les cires ou les polymères. Les polymères sont en particulier ceux décrits dans le document WO-A-93/01797 déposé au nom du demandeur comme le copolymère de méthacrylate de N, N-di méthylaminoéthylelméthacrylate de stéaryle, le copolymère acylate de dodécyle/ acide méthacrylique, le copolymère acrylate de dodécyle/méthacrylate de stéaryle/acide méthacrylique.

Le gélifiant ou le mélange de gélifiants peut représenter de 1 % à 15 % en poids et de préférence de 2 % à 10 % en poids sauf lorsque ce gélifiant représente de la vaseline ; dans ce dernier cas on peut utiliser jusqu'à 50 % en poids du gel.

Si nécessaire, on peut ajouter au gel huileux un ou plusieurs agents tensioactifs lipophiles ayant une valeur de HLB (équilibre hydrophile/lipophile) > à 8 et en général allant de 9 à 12.

La quantité de tensioactifs peut varier de 1 % à 15 % du poids total du gel et de préférence choisie de 3 % à 10 %.

Comme agents tensioactifs, on peut citer des alcools oxyéthylénés comme l'octyldodécanol oxyéthyléné à 25 moles d'oxyde d'éthylène, des oléates de sorbitol ou de sorbitane éventuellement oxyéthylénés comme le septa-oléate de sorbitol oxyéthyléné à 40 moles d'oxyde d'éthylène et le tri-oléate de sorbitane oxyéthyléné à 20 moles d'éthylène.

Les gels huileux de l'invention peuvent, en outre, contenir divers ingrédients classiquement utilisés dans les domaines cosmétique et/ou dermatologique. Ces ingrédients peuvent consister en des actifs hydrophiles, des actifs lipophiles, des parfums, des conservateurs, des matières colorantes, des cires, des argiles ou encore des agents de texture comme des agents pulvérulents autres que les particules exfoliantes. Ces ingrédients additionnels sont utilisés en quantité habituelle.

Comme actifs hydrophiles, on peut citer les polyols tels que le propylène glycol, la glycérine, le sorbitol. Comme actifs lipophiles, on peut citer les huiles essentielles ou les huiles démaquillantes comme le palmitate d'éthyl-2 hexyle.

La composition gélifiée de l'invention est quasiment anhydre. Elle peut être transparente ou non selon les ingrédients qui la constituent. Elle s'élimine facilement à l'eau.

De façon avantageuse, le gel huileux de l'invention est un gel à usage cosmétique et/ou dermatologique.

Aussi, l'invention a également pour objet un gel gommant pour visage ainsi qu'un gel gommant pour corps humain consistant en un gel tel que défini précédemment. Lorsque ce gel est destiné au nettoyage et/ou gommage du visage, il contient de préférence de 1 % à 4 % en poids de particules par rapport au poids total du gel. Lorsque ce gel est destiné au nettoyage et/ou gommage du corps humain, il contient avantageusement de 5 % à 12 % en poids de particules exfoliantes par rapport au poids total du gel.

L'invention a aussi pour objet un procédé pour nettoyer et/ou gommer la peau du corps et/ou du visage, y compris le cou, consistant à appliquer sur la peau un gel tel que défini précédemment, à masser la peau avec ce gel pour éliminer les cellules mortes puis à rincer la peau, généralement à l'eau en vue d'éliminer ces cellules mortes et les particules.

L'invention a aussi pour objet l'utilisation du gel défini précédemment pour nettoyer et/ou gommer la peau du visage, du cou et/ou du corps humain.

Le nettoyage du corps humain concerne aussi bien les jambes, que les bras, le dos et le ventre, les coudes, les pieds et les genoux.

Compte-tenu des propriétés non irritantes du gel de l'invention, celui-ci peut être utilisé quotidiennement même par les personnes à peau très sèche.

Les exemples qui suivent sont donnés à titre illustratif en vue de mieux faire comprendre l'invention. Les quantités sont données en % en poids.

### Exemple 1 : Il s'agit d'un produit exfoliant pour le corps, pour peaux sensibles, de consistance cireuse, d'une viscosité de 1300 cp (1,3 Pa.s).

| | | |
|---|---|---|
| (a) - | Huile de vaseline | qsp 100 % |
| (b) - | Octyldodécanol | 26 % |
| (c) - | Cire de polyéthylène | 9 % |
| (d) - | Septaoléate de sorbitol oxyéthyléné (40 OE) | 4 % |
| (e) - | Trioléate de sorbitane oxyéthyléné (20 OE) | 6 % |
| (f) - | Poudre de polyéthylène | 5 % |
| (g) - | Parfum | 0,6 % |
| (h) - | Colorant | 0.0001% |
| Mode opératoire : Mélanger les constituants a, b, c, e et h à 95 °C. Homogénéiser jusqu'à 60 °C puis ajouter d. A 40 °C introduire f et le parfum. | | |

### Exemple 2 : Il s'agit d'un gel huileux exfoliant translucide destiné au corps, d'une viscosité de 13500 cp (13,5 Pa.s)

| *Phase A* | | |
|---|---|---|
| (a) - | Huile de vaseline | qsp 100 % |
| (b) - | Palmitate d'éthyl-2-hexyle | 30 % |
| (c) - | Huile de jojoba | 1 % |
| (d) - | C₁₁-C₁₃ Isoparaffine | 10 % |
| (e) - | Copolymère de méthacrylate de N, N-di -méthylaminoéthyle/méthacrylate de stéaryle | 1,25 % |
| (f) - | Copolymère acylate de dodécyle/acide méthacrylique | 0,75 % |
| (g) - | Copolymère acrylate de dodécyle/méthacrylate de stéaryle/acide méthacrylique | 0,25 % |

| *Phase B* | | |
|---|---|---|
| (h) - | Septaoléate de sorbitol oxyéthyléné (40 OE) | 4 % |
| (i) - | Octyldodecanol oxyéthyléné (25 OE) | 4,5 % |
| (j) - | Eau | 4,5 % |
| (k) - | Glycérine | 6 % |

| *Phase C* | | |
|---|---|---|
| (l) - | Poudre de polyéthylène | 5 % |
| (m) - | Colorant | 0,0002 % |
| (n) - | Parfum | 0,6 % |
| Mode opératoire : *Phase A* : A 60 °C mélanger a, b, e, f et g. Rabaisser la température à 40 °C et introduire c et d. *Phase B* : A 80 °C mélanger h, i, j, et k. Mettre *B* dans *A*, puis à 40 °C introduire I, m et n. | | |

### Exemple 3 : Il s'agit d'un gel exfoliant visage pour les peaux très sèches, d'une viscosité 500 cp (0,5 Pa.s).

| | | |
|---|---|---|
| (a) - | Huile de vaseline | qsp 100 % |
| (b) - | Isopropyl palmitate | 37 % |
| (c) - | Vaseline (cire) | 15 % |
| (d) - | Propylène carbonate | 0.1 % |
| (e) - | Hectorite modifiée par chlorure de di-stéaryl di-méthyl ammonium (argile) | 0,25 % |
| (f) - | Ozokérite (cire) | 10 % |
| (g) - | Septaoléate de sorbitane oxyéthyléné (40 OE) | 2 % |
| (h) - | Poudre de polyéthylène | 2 % |
| (i) - | Conservateurs | 0,35 % |
| Mode opératoire : Mélanger à 90 °C a, b, c, d, e et f. Puis abaisser la température à 60 °C et ajouter g et les conservateurs. A 40 ° C disperser h. | | |

### Exemple 4 : Il s'agit d'un produit exfoliant pour les talons, les coudes, les plantes des pieds, ... (zones corporelles où la couche cornée est très épaisse).

| | | |
|---|---|---|
| (a) - | Huile de vaseline | qsp 100 % |
| (b) - | lsopropyl palmitate | 32 % |
| (c) - | Vaseline | 15 % |
| (d) - | Propylène carbonate | 0,1 % |
| (e) - | Hectorite modifiée par chlorure de di-stéaryl di-méthyl ammonium | 0,25 % |
| (f) - | Ozokérite | 10 % |
| (g) - | Septaoléate de sorbitane oxyéthyléné (40 OE) | 1 % |
| (h) - | Poudre de coque de noix | 10 % |
| (i) - | Conservateur | 0,05 % |
| Mode opératoire : identique à celui de l'exemple 3. | | |

Tous ces gels huileux sont du type produit "2 en 1"; ils assurent un gommage et un soin de la peau.

Le gel corporel de l'exemple 1 a été testé par 61 femmes. Ce gel a été fortement apprécié, notamment pour ses propriétés cosmétiques et surtout pour ses propriétés gommantes.

Plus précisément, les effets les plus remarquables sont :
- l'effet adoucissant (immédiat : 98 %, et à long terme : 79 %),
- l'effet gommant (immédiat : 84 %, polissant dans le temps : 75 %)
- l'effet nettoyant (74 %)
- l'effet hydratant est également noté par les 2/3 des femmes et surtout, 29 femmes (sur 42 concernées par le problème) trouvent que le produit évite les desquamations.

Concernant toujours l'aspect "2 en 1", il est important de voir que 82 % des femmes n'ont pas jugé utile d'appliquer un lait après rinçage. En outre, le gel se rince facilement pour 82 % des femmes.

Les aspects cosmétiques sont également très bons compte-tenu de l'aspect nouveau du produit et de son mode d'emploi.

L'application est très facile (92 %) quelle que soit la méthode choisie, toutefois, l'utilisation sur peau humide a été plus souvent employée.

La texture a été appréciée par 89 % des utilisatrices et une agréable sensation de bien-être a été perçue par 74 % des femmes.

En outre, 15/18 femmes ayant choisi la méthode sur peau sèche estiment que la texture renforce l'efficacité du gel.

Après rinçage, 41/61 femmes notent la présence d'un film résiduel, qui n'est en aucun cas gênant bien au contraire, puisqu'il est associé à un film protecteur pour 85 % d'entre elles.

Le confort est excellent (95 %) et la satisfaction globale est bonne (72 %), la plupart du temps en raison de l'efficacité du produit.

En conclusion, il s'agit d'un produit très attrayant pour les femmes et visiblement très efficace. Globalement, le concept "2 en 1" exfofiant/hydratant est bien accepté.

Par ailleurs, près de 100 % de ces femmes ont trouvé que la peau était, de façon spectaculaire, plus douce et satinée, immédiatement mais également cinq semaines après le nettoyage. De plus, 69 % de ces femmes ont trouvé au gel un rôle préventif contre les desquamations.

En conclusion, le gel de l'invention associe une action gommante effective à un soin indéniable.

## Revendications

1. Gel huileux gommant, **caractérisé en ce qu'**il contient une dispersion homogène de particules exfoliantes, que sa viscosité est ajustée à une valeur allant de 500mPa.s à 13500mPa.s, et **en ce qu'**il contient un gélifiant d'huile en une quantité suffisante pour assurer une dispersion homogène des particules exfoliantes, une viscosité de 500 mPa.s correspondant à la viscosité d'une composition ayant la composition suivante :
| | | |
|---|---|---|
| (a) - | huile de vaseline qsp | 100 % |
| (b) - | isopropyl palmitate | 37 % |
| (c) - | vaseline (cire) | 15 % |
| (d) - | propylène carbonate | 0,1 % |
| (e) - | hectorite modifiée | 0,25 % |
| (f) - | ozokérite (cire) | 10 % |
| (g) - | septaoléate de sorbitane oxyéthyléné (40 OE) | 2 % |
| (h) - | poudre de polyéthylène | 2 % |
| (i) - | conservateurs | 0,35 % |
qui est préparée en mélangeant à 90°C les composants (a), (b), (c), (d), (e) et (f) suivie par un abaissement de la température à 60°C et l'ajout de (g) et des conservateurs et la dispersion dé (h) à 40°C et une viscosité de 13500 mPa.s correspondant à la viscosité d'une composition obtenue par la préparation d'une phase A constituée par :
| | | |
|---|---|---|
| (a) - | huile de vaseline qsp | 100 % |
| (b) - | palmitate d'éthyl-2-hexyle | 30 % |
| (c) - | huile de jojoba | 1 % |
| (d) - | C11-C13 isoparaffine | 10 % |
| (e) - | copolymère de méthacrylate de N, N-di-méthylaminoéthyle/méthacrylate de stéaryle | 1,25 % |
| (f) - | copolymère acylate de dodécyle/acide méthacrylique | 0,75 % |
| (g) - | copolymère acrylate de dodécyle/méthacrylate de stéaryle/acide méthacrylique | 0,25 % |
obtenue par mélange de (a), (b), (e), (f) et (g) à 60°C, puis abaissement de la température à 40°C et introduction de (c) et (d), d'une phase B constituée par :
| | | |
|---|---|---|
| (h) - | septaoléate de sorbitol oxyéthyléné (40 OE) | 4 % |
| (i) - | octyldodecanol oxyéthyléné (25 OE) | 4,5 % |
| (j) - | eau | 4,5 % |
| (k) - | glycérine | 6 % |
par mélange des différents éléments à 80°C,
puis introduction de la phase B dans la phase A et introduction des éléments (l), (m) et (n) d'une phase C :
| | | |
|---|---|---|
| (l) - | poudre de polyéthylène | 5 % |
| (m)- | colorant | 0,0002 % |
| (n) - | parfum | 0,6 % |
à 40°C.

2. Gel selon la revendication 1, **caractérisé en ce que** les particules sont des particules en matériau organique polymérique.

3. Gel selon la revendication précédente 1 ou 2, **caractérisé en ce que** les particules sont des particules de polyéthylène.

4. Gel selon l'une quelconque des revendications précédentes, **caractérisé en ce que** les particules ont un diamètre allant de 100µm à 500µm.

5. Gel selon l'une quelconque des revendications précédentes, **caractérisé en ce que** les particules ont un diamètre allant de 150µm à 300µm.

6. Gel selon l'une quelconque des revendications précédentes, **caractérisé en ce qu'**il contient moins de 10 % d'une phase aqueuse par rapport au poids total du gel.

7. Gel selon l'une quelconque des revendications précédentes, **caractérisé en ce qu'**il contient de 50 % à 95 % en poids d'huile par rapport au poids total du gel.

8. Gel selon l'une quelconque des revendications précédentes, **caractérisé en ce qu'**il contient de 70 % à 85 % en poids d'huile par rapport au poids total du gel.

9. Gel selon l'une quelconque des revendications précédentes, **caractérisé en ce qu'**il contient de 0,5 % à 15 % en poids de particules par rapport au poids total du gel.

10. Gel selon l'une quelconque des revendications précédentes, **caractérisé en ce qu'**il contient de 1 % à 12 % en poids de particules par rapport au poids total du gel.

11. Gel selon l'une quelconque des revendications précédentes, **caractérisé en ce qu'**il contient, en outre, au moins un adjuvant choisi parmi les gélifiants, les agents tensio-actifs, les actifs hydrophiles, les actifs lipophiles, les parfums, les conservateurs, les matières colorantes, les cires, les argiles.

12. Gel selon l'une quelconque des revendications précédentes, **caractérisé en ce qu'**il contient, en outre, de 1 % à 15 % en poids de gélifiant par rapport au poids total du gel.

13. Gel selon l'une quelconque des revendications précédentes, **caractérisé en ce qu'**il consiste en gel cosmétique et/ou dermatologique.

14. Gel gommant pour visage, **caractérisé en ce qu'**il consiste en un gel selon l'une quelconque des revendications précédentes.

15. Gel selon la revendication 14, **caractérisé en ce qu'**il contient de 1 % à 4 % en poids de particules par rapport au poids total du gel.

16. Gel gommant pour corps humain, **caractérisé en ce qu'**il consiste en un gel selon l'une quelconque des revendications 1 à 13.

17. Gel selon la revendication 16, **caractérisé en ce qu'**il contient de 5 % à 12 % en poids de particules par rapport au poids total du gel.

18. Procédé pour nettoyer et/ou gommer la peau du corps, du cou et/ou du visage, **caractérisé en ce qu'**il consiste à appliquer sur la peau un gel selon l'une quelconque des revendications précédentes, à masser la peau avec le gel pour éliminer les cellules mortes et à rincer la peau.

19. Utilisation du gel selon l'une quelconque des revendications 1 à 17, pour nettoyer et/ou gommer la peau du visage, du cou et/ou du corps humain.

## Patentansprüche

1. Öliges Peeling-Gel, **dadurch gekennzeichnet, dass** es eine homogene Dispersion von abrasiven Partikeln enthält, dass seine Viskosität auf einen Wert im Bereich von 500 bis 13500 mPa·s eingestellt ist und dass es einen Gelbildner für Öl in einer Menge enthält, die ausreicht, um eine homogene Dispersion der abrasiven Partikel zu gewährleisten, wobei eine Viskosität von 500 mPa·s der Viskosität einer Zusammensetzung entspricht, die wie folgt zusammengesetzt ist:
| | | |
|---|---|---|
| (a) - | Vaselinöl ad | 100 % |
| (b) - | Isopropylpalmitat | 37 % |
| (c) - | Vaseline (Wachs) | 15 % |
| (d) - | Propylencarbonat | 0,1 % |
| (e) - | modifizierter Hectorit | 0,25 % |
| (f) - | Ozokerit (Wachs) | 10 % |
| (g) - | ethoxyliertes Sorbitan-septaoleat | 2 % |
| | (40 EO-Einheiten) | |
| (h) - | Polyethylenpulver | 2 % |
| (i) - | Konservierungsstoffe | 0,35 % |
und hergestellt ist durch Mischen der Bestandteile (a), (b), (c), (d), (e) und (f) bei 90 °C, anschließendes Absenken der Temperatur auf 60 °C und Zufügen von (g) und der Konservierungsstoffe und Dispergieren von (h) bei 40 °C,
und wobei eine Viskosität von 13500 mPa·s der Viskosität einer Zusammensetzung entspricht, die erhalten ist durch Herstellung einer Phase A folgender Zusammensetzung:
| | | |
|---|---|---|
| (a) - | Vaselinöl ad | 100 % |
| (b) - | 2-Ethylhexylpalmitat | 30 % |
| (c) - | Jojobaöl | 1 % |
| (d) - | C₁₁₋₁₃-Isoparaffine | 10 % |
| (e) - | N,N-Dimethylaminoethylmethacrylat-Stearylmethacrylat-Copolymer | 1,25 % |
| (f) - | Dodecylacylat-Methacrylsäure-Copolymer | 0,75 % |
| (g) - | Dodecylacrylat-Stearylmethacrylat-Methacrylsäure-Copolymer | 0,25 %, |
die erhalten ist durch Mischen von (a), (b), (e), (f) und (g) bei 60 °C, anschließendes Absenken der Temperatur auf 40 °C und Zufügen von (c) und (d),
Herstellung einer Phase B folgender Zusammensetzung:
| | | |
|---|---|---|
| (h) - | ethoxyliertes Sorbit-septaoleat | |
| | (40 EO-Einheiten) | 4 % |
| (i) - | ethoxyliertes Octyldocecanol (25 EO-Einheiten) | 4,5 % |
| (j) - | Wasser | 4,5 % |
| (k) - | Glycerin | 6 %, |
die durch Mischen der verschiedenen Bestandteile bei 80 °C erhalten ist,
Einführen der Phase B in die Phase A und Einführen der Bestandteile (l), (m) und (n) einer Phase C:
| | | |
|---|---|---|
| (l) - | Polyethylenpulver | 5 % |
| (m) - | Färbemittel | 0,0002 % |
| (n) - | Parfum | 0,6 % |
bei 40 °C.

2. Gel nach Anspruch 1, **dadurch gekennzeichnet, dass** die Partikel aus einem organischen Polymermaterial bestehen.

3. Gel nach Anspruch 1 oder 2, **dadurch gekennzeichnet, dass** die Partikel Polyethylenpartikel sind.

4. Gel nach einem der vorhergehenden Ansprüche, **dadurch gekennzeichnet, dass** die Partikel einen Durchmesser von 100 bis 500 µm aufweisen.

5. Gel nach einem der vorhergehenden Ansprüche, **dadurch gekennzeichnet, dass** die Partikel einen Durchmesser von 150 bis 300 µm aufweisen.

6. Gel nach einem der vorhergehenden Ansprüche, **dadurch gekennzeichnet, dass** es weniger als 10 % an wässeriger Phase, bezogen auf das Gesamtgewicht des Gels, enthält.

7. Gel nach einem der vorhergehenden Ansprüche, **dadurch gekennzeichnet, dass** es 50 bis 95 Gew.-% Öl, bezogen auf das Gesamtgewicht des Gels, enthält.

8. Gel nach einem der vorhergehenden Ansprüche, **dadurch gekennzeichnet, dass** es 70 bis 85 Gew.-% Öl, bezogen auf das Gesamtgewicht des Gels, enthält.

9. Gel nach einem der vorhergehenden Ansprüche, **dadurch gekennzeichnet, dass** es 0,5 bis 15 Gew.-% Partikel, bezogen auf das Gesamtgewicht des Gels, enthält.

10. Gel nach einem der vorhergehenden Ansprüche, **dadurch gekennzeichnet, dass** es 1 bis 12 Gew.-% Partikel, bezogen auf das Gesamtgewicht des Gels, enthält.

11. Gel nach einem der vorhergehenden Ansprüche, **dadurch gekennzeichnet, dass** es ferner mindestens einen Hilfsstoff enthält, der ausgewählt ist unter Gelbildnern, grenzflächenaktiven Mitteln, hydrophilen Wirkstoffen, Wirkstoffen, Parfums, Konservierungsstoffen, Färbemitteln, Wachsen und Tonen.

12. Gel nach einem der vorhergehenden Ansprüche, **dadurch gekennzeichnet, dass** es ferner 1 bis 15 Gew.-% Gelbildner, bezogen auf das Gesamtgewicht des Gels, enthält.

13. Gel nach einem der vorhergehenden Ansprüche, **dadurch gekennzeichnet, dass** es aus einem kosmetischen und/oder dermatologischen Gel besteht.

14. Peeling-Gel für das Gesicht, **dadurch gekennzeichnet,**
**dass** es aus einem Gel nach einem der vorhergehenden Ansprüche besteht.

15. Gel nach Anspruch 11, **dadurch gekennzeichnet, dass** es 1 bis 4 Gew.-% Partikel, bezogen auf das Gesamtgewicht des Gels, enthält.

16. Peeling-Gel für den menschlichen Körper, **dadurch gekennzeichnet, dass** es aus einem Gel nach einem der Ansprüche 1 bis 13 besteht.

17. Gel nach Anspruch 13, **dadurch gekennzeichnet, dass** es 5 bis 12 Gew.-% Partikel, bezogen auf das Gesamtgewicht des Gels, enthält.

18. Verfahren zum Reinigen und/oder zum Peeling der Haut des Körpers, des Halses und/oder des Gesichts, **dadurch gekennzeichnet, dass** es darin besteht, auf die Haut ein Gel nach einem der vorhergehenden Ansprüche aufzutragen, die Haut mit dem Gel zu massieren, um abgestorbene Zellen zu entfernen, und die Haut abzuspülen.

19. Verwendung des Gels nach einem der Ansprüche 1 bis 17 zur Reinigung und/oder zum Peeling der Haut des Gesichts, des Halses und/oder des menschlichen Körpers.

## Claims

1. Oily peeling gel, **characterized in that** it contains a homogeneous dispersion of exfoliating particles, **in that** its viscosity is adjusted to a value ranging from 500 mPa.s to 13 500 mPa.s and **in that** it contains an oil-gelling agent in a quantity sufficient to provide a homogeneous dispersion of the exfoliating particles, a viscosity of 500 mPa.s corresponding to the viscosity of a composition having the following composition:
| | | |
|---|---|---|
| (a) - | petroleum jelly qs | 100 % |
| (b) - | isopropyl palmitate | 37 % |
| (c) - | vaseline (wax) | 15 % |
| (d) - | propylene carbonate | 0.1 % |
| (e) - | modified hectorite | 0.25 % |
| (f) - | ozokerite (wax) | 10 % |
| (g) - | oxyethylenated sorbitan | |
| | septaoleate (40 EO) | 2 % |
| (h) - | polyethylene powder | 2 % |
| (i) - | preservatives | 0.35 % |
which is prepared by mixing the components (a), (b), (c) , (d), (e) and (f) at 90°C, followed by a reduction in the temperature to 60°C and the addition of (g) and the preservatives and the dispersion of (h) at 40°C, and a viscosity of 13 500 mPa.s corresponding to the viscosity of a composition obtained by the preparation of a phase A composed of:
| | | |
|---|---|---|
| (a) - | petroleum jelly qs | 100 % |
| (b) - | 2-ethylhexyl palmitate | 30 % |
| (c) - | jojoba oil | 1 % |
| (d) - | C₁₁-C₁₃ isoparaffin | 10 % |
| (e) - | N,N-dimethylaminoethyl methacrylate/stearyl methacrylate copolymer | 1.25 % |
| (f) - | dodecyl acrylate/methacrylic acid copolymer | 0.75 % |
| (g) - | dodecyl acrylate/stearyl methacrylate/methacrylic acid copolymer | 0.25 % |
obtained by mixing (a), (b), (e), (f) and (g) at 60°C, then reducing the temperature to 40°C and introducing (c) and (d),
of a phase B composed of:
| | | |
|---|---|---|
| (h) - | oxyethylenated sorbitol septaoleate (40 EO) | 4 % |
| (i) - | oxyethylenated octyldodecanol (25 EO) | 4.5 % |
| (j) - | water | 4.5 % |
| (k) - | glycerine | 6 % |
by mixing the various components at 80°C,
then introduction of the phase B into the phase A and introduction of the components (l), (m) and (n) of a phase C:
| | | |
|---|---|---|
| (l) - | polyethylene powder | 5 % |
| (m) - | colorant | 0.0002 % |
| (n) - | perfume | 0.6 % |
at 40°C.

2. Gel according to Claim 1, **characterized in that** the particles are particles made of polymeric organic material.

3. Gel according to the preceding Claim 1 or 2, **characterized in that** the particles are polyethylene particles.

4. Gel according to any one of the preceding claims, **characterized in that** the particles have a diameter ranging from 100 µm to 500 µm.

5. Gel according to any one of the preceding claims, **characterized in that** the particles have a diameter ranging from 150 µm to 300 µm.

6. Gel according to any one of the preceding claims, **characterized in that** it contains less than 10% of an aqueous phase relative to the total weight of the gel.

7. Gel according to any one of the preceding claims, **characterized in that** it contains from 50% to 95% by weight of oil relative to the total weight of the gel.

8. Gel according to any one of the preceding claims, **characterized in that** it contains from 70% to 85% by weight of oil relative to the total weight of the gel.

9. Gel according to any one of the preceding claims, **characterized in that** it contains from 0.5% to 15% by weight of particles relative to the total weight of the gel.

10. Gel according to any one of the preceding claims, **characterized in that** it contains from 1% to 12% by weight of particles relative to the total weight of the gel.

11. Gel according to any one of the preceding claims, **characterized in that** it contains, in addition, at least one adjuvant chosen from gelling agents, surfactants, hydrophilic active agents, lipophilic active agents, perfumes, preservatives, colorants, waxes, clays.

12. Gel according to any one of the preceding claims, **characterized in that** it contains, in addition, from 1% to 15% by weight of gelling agent relative to the total weight of the gel.

13. Gel according to any one of the preceding claims, **characterized in that** it consists of a cosmetic and/or dermatological gel.

14. Peeling gel for the face, **characterized in that** it consists of a gel according to any one of the preceding claims.

15. Gel according to Claim 14, **characterized in that** it contains from 1% to 4% by weight of particles relative to the total weight of the gel.

16. Peeling gel for the human body, **characterized in that** it consists of a gel according to any one of Claims 1 to 13.

17. Gel according to Claim 16, **characterized in that** it contains from 5% to 12% by weight of particles relative to the total weight of the gel.

18. Process for cleaning and/or scrubbing the skin of the body, of the neck and/or of the face, **characterized in that** it consists in applying to the skin a gel according to any one of the preceding claims, in rubbing the skin with the gel in order to remove the dead cells and in rinsing the skin.

19. Use of the gel according to any one of Claims 1 to 17, for cleaning and/or scrubbing the skin of the face, of the neck and/or of the human body.
